# EUROPEAN PATENT SPECIFICATION

(11) **EP 2 238 910 B1**
(45) Date of publication and mention of the grant of the patent: **29.07.2015**
(21) Application number: 09290259.2
(22) Date of filing: 08.04.2009
(51) Int. Cl.: A61B 7/02

(54) **Monitoring device**
Überwachungsvorrichtung
Dispositif de surveillance

(43) Date of publication of application: 13.10.2010
(73) Proprietor: ALCATEL LUCENT, 92100 Boulogne-Billancourt (FR)
(72) Inventor: Scanlon, Patricia, Portobello, 8 Dublin (IE)
(74) Representative: Berthier, Karine

(56) References cited:
- WO-A-2009/109917
- US-A1- 2006 129 067
- US-A1- 2007 010 722
- US-A1- 2008 139 893

## Description

### Background

This section introduces aspects that may be helpful to facilitating a better understanding of the inventions. Accordingly, the statements of this section are to be read in this light and are not to be understood as admissions about what is prior art or what is not the prior art.

The present invention relates to monitoring devices, including but not limited to devices for auscultation.

Auscultation is the term used for listening to the internal sounds of the body. This is currently generally done using a stethoscope. It is a skill that requires substantial clinical experience, and good listening skills. Doctors listen to three main organs/organ systems during auscultation: the heart, lungs, and the gastrointestinal system. When auscultating the heart, doctors listen for abnormal sounds that may indicate serious conditions such as heart murmurs, gallops, and other extra sounds coinciding or correlated with heartbeats. Heart rate may also be noted based on auscultation. When listening to lungs, breath sounds such as wheezes and crackles can be identified that may indicate respiratory problems. Auscultation of the abdomen can also indicate problems with the gastrointestinal system.

Home tele-monitoring services allow patients' vital signs data to be remotely monitored by medical professionals. The primary benefit of tele-monitoring is better management of medical conditions, which means people stay healthier.

A prior art apparatus for sensing and analysing body sounds is known from US 2008/0139893. A prior art wearable auscultation system is known from US 2006/0129067

### Summary

An electronic stethoscope will allow patients to perform auscultation in the home. This allows for the recording of auscultation sounds and the transmission of these to medical professionals for the purpose of telemedicine, or remote diagnosis. The quality of the data acquired and transmitted is vital to the success of such a tele-monitoring service. Auscultation is an acquired expert medical skill and it is therefore not possible to acquire good quality, consistent auscultation data from patients using stethoscopes themselves.

Various embodiments provide a monitoring device capable of acquiring consistent, good quality auscultation sounds without the need for manual data acquisition by patients, or the need for expert help or intervention.

According to a first embodiment, there is provided a monitoring device as recited by Claim 1.

By providing the sensors in a garment, it is possible to ensure correct alignment and consistent contact of the sensors with the points on the wearer's body that permit monitoring, without the input of a medical professional. Moreover, passive and continuous monitoring of the wearer are possible, since the garment can be worn at all times, including, for example, when sleeping.

Preferably, the sensors are arranged to detect body surface vibrations for auscultation monitoring.

Preferably, the array includes sensors located at locations corresponding to cardiac and/or pulmonary auscultation sites of the wearer.

Preferably, the array comprises at least sensors arranged at locations corresponding to the cardiac and pulmonary auscultation sites on the anterior chest and corresponding to the pulmonary auscultation sites on the posterior chest.

This is particularly advantageous, since it is possible for auscultation to be performed at auscultation sites on the wearer's back, which would previously have been out of reach of the wearer and required assistance from another person.

The array may comprise a greater number of sensors than auscultation sites and the control means may be arranged to analyse data acquired at the sensors to determine which sensors are aligned with predetermined cardiac and/or pulmonary auscultation sites.

In embodiments having a greater number of sensors than auscultation sites, it may be possible to compensate for incorrect positioning of the vest on a wearer, or to accommodate for variations in the positioning of auscultation sites from one wearer to another. The array may cover substantially the entire front and/or rear portions of the garment or substantial contiguous areas on the front and/or rear of the garment, e.g., 30% or more of the areas of the front and/or rear body portions of the garment or even 50% or more of said areas. Alternatively, the array may comprise a plurality of sensor clusters, wherein each cluster is located at a respective auscultation location on the wearer's chest.

The control means may be arranged to determine which sensors are aligned with the predetermined auscultation sites using matched filters.

The control means may be arranged to switch off any sensors that are not aligned with the predetermined auscultation sites.

The sensors may comprise microphones, accelerometers, piezoelectric elements, acoustic stethoscopes or electronic stethoscopes.

A combination of these sensors is possible. Alternatively, all of the sensors may be of the same type.

The sensors may be provided with noise amplification and/or cancellation means.

The garment may further comprise one or more additional sensors selected from accelerometers, gyroscope sensors, resistance sensors, temperature sensors, ECGs or blood pressure sensors, or other types of sensor.

By providing sensors in addition to the sensors arranged to detect body surface vibrations, it is possible to build a more complete picture of the wearer's status during auscultation reading using vital or contextual information.

Preferably, the garment is a vest, e.g., a garment worn over the torso and not having sleeves.

According to a further embodiment there is provided a monitoring system comprising a monitoring device as detailed above and a computer, wherein the control unit of the garment is arranged to be connected to the computer for the download of data from the sensors.

According to another embodiment, there is provided a method of monitoring the bodily functions of a wearer using the monitoring device or the monitoring system detailed above.

Advantages of embodiments may include: the ability to continuously monitor a patient; the ability of an individual to take pulmonary auscultation readings on the posterior chest unaided; the consistent acquisition of data without the need for patient co-operation or expert intervention; the acquisition of the best auscultation signal for each auscultation site; the provision of contextual or vital information, which aids in diagnosis and/or the filtering of irrelevant data; and the ability to perform tele-monitoring of heart/lung/bowel sounds, which would not be possible or reliable using existing devices.

The term "corresponding to" or similar should be construed as meaning not only precisely positioned but also positioned so that when the garment is worn the sensor is sufficiently close to an auscultation site that a measurable signal is detected by the sensor.

### Description of the Drawings

Embodiments will now be described, by way of example only, with reference to the accompanying drawings:
Figure 1 shows a monitoring device according to an embodiment;
Figure 2 shows a possible sensor array for the vest of Figure 1;
Figure 3 shows an alternative possible sensor array for the vest of Figure 1;
Figure 4 shows pulmonary auscultation sites on the chest wall;
Figure 5 shows pulmonary auscultation sites on the back wall; and
Figure 6 shows cardiac auscultation sites on the chest wall.

### Detailed Description of Embodiments

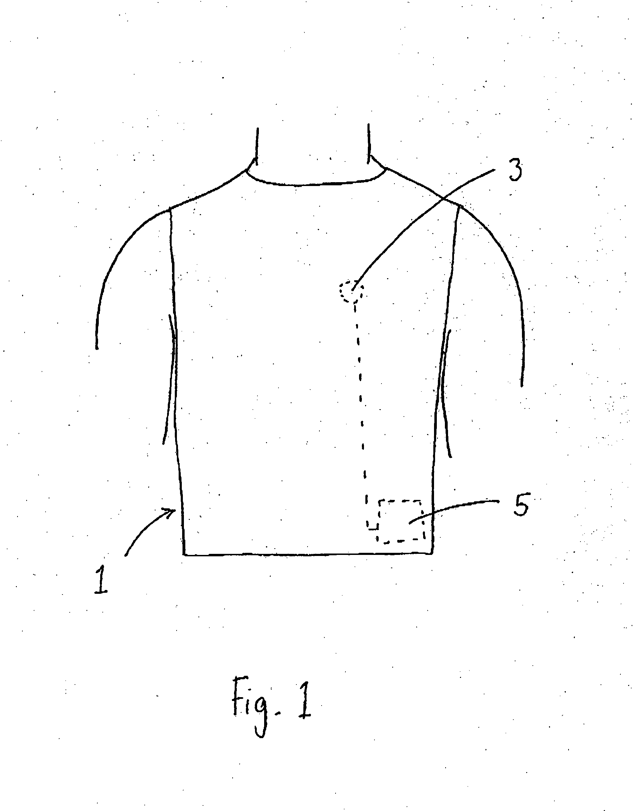

Referring to Figure 1, there is shown a monitoring device according to an embodiment, which comprises a vest 1 that is provided with at least one sensor array. In Figure 1, a single sensor 3 is shown, which is connected to a control means 5. It should be appreciated that whilst a vest is shown, numerous alternative garment types may be used, including, for example, bibs/tabards, shirts or garments with sleeves. Any garment may be used that is capable of maintaining the sensor array in contact with the wearer's body, or sufficiently close thereto that a monitorable signal is detectable at the sensor or sensors.

The vest is preferably flexible and may be elasticised such that it conforms closely to the body of the wearer. It is preferably lightweight and may be made of any suitable materials, including but not limited to cotton or polyester or mixes thereof, It is provided with sensors at both the front and the rear, however, in alternative arrangements may comprise only front or rear mounted sensors depending on application.

The sensors are arranged to detect bodily sounds. The sensors preferably detect bodily sounds by measuring body surface vibrations. The sensors may comprise microphones, accelerometers, piezoelectric elements, acoustic stethoscopes or electronic stethoscopes. Combinations of the different sensor types may be incorporated into the same vest. Any suitable known sensor type may be incorporated into the vest.

The sensors may be fixed to the vest in any suitable manner. The sensors may, for example, be stitched into the vest. Alternatively, the sensors may be adhered to a surface of the vest. The sensors may be permanently attached to the vest or may be removably attached, to allow for the vest to be laundered.

The vest may be arranged such that the sensors may be urged against the skin of the wearer with a consistent pressure and so as to prevent lateral movement of the sensors against the skin, which could introduce unwanted scraping sounds.

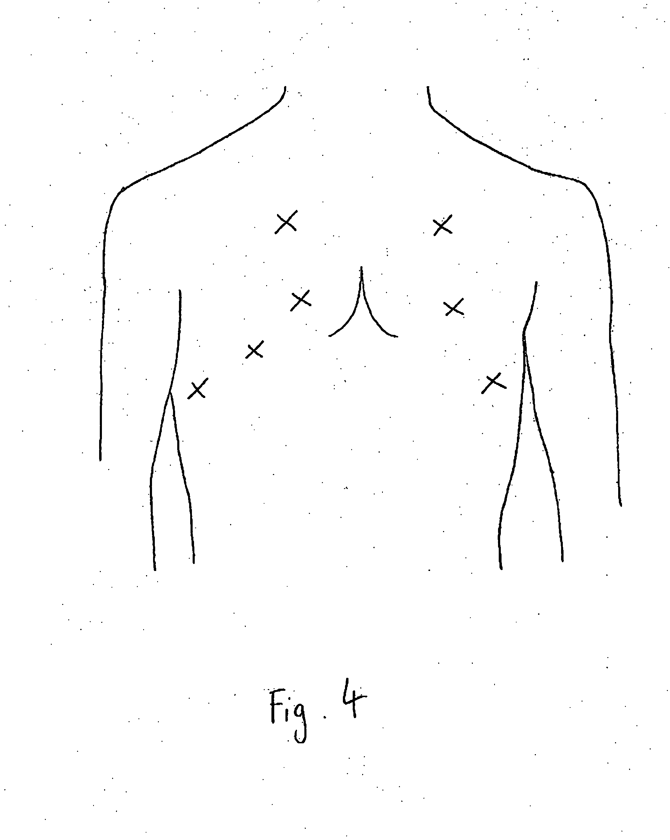

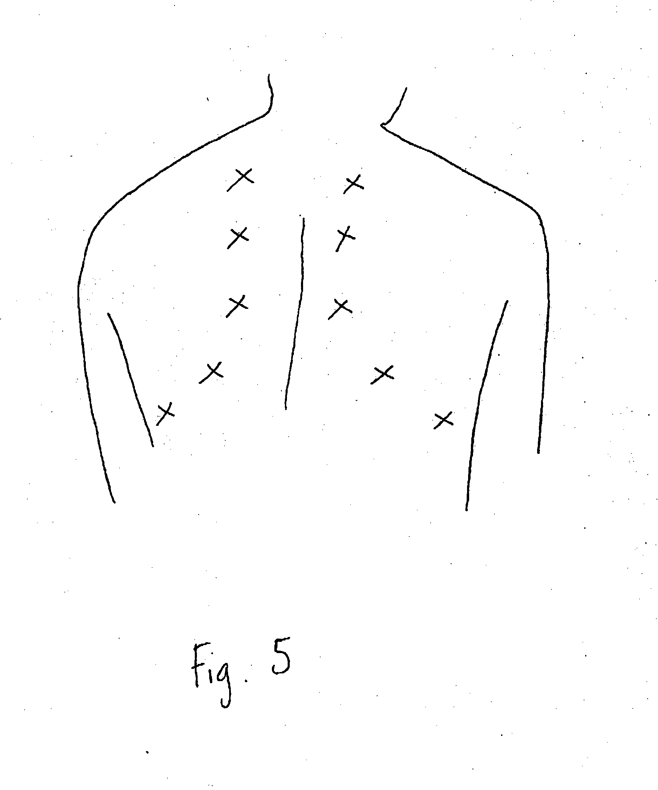

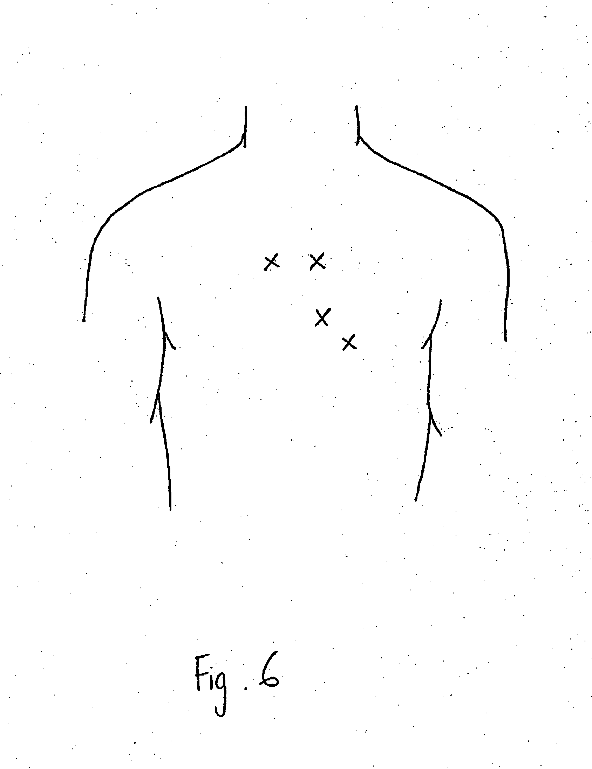

There are believed to be about 12 locations for pulmonary auscultation on the anterior chest (chest wall) and about 14 locations for pulmonary auscultation on the posterior chest (back wall), a number of which are shown marked with crosses in Figures 4 and 5 respectively. There are about 4 locations for cardiac auscultation on the chest wall, as shown marked with crosses in Figure 6.

The sensors are preferably arranged to allow for detection of body surface vibrations in all of the pulmonary and cardiac auscultation locations.

There may be provided an array of sensors, which comprises an individual sensor at each location on the vest that corresponds, in use, to a respective auscultation location on the wearer's chest. With such an arrangement it is possible to tailor a vest to a particular wearer and minimise the number of sensors required. The vest can, for example, be configured for a particular wearer by a medical professional after which the vest can be used without the aid of a medical professional. The control means is preferably arranged to control the sensors, wherein a number of the sensors can be shut off with only the sensors that cover or lie next to or adjacent to the areas of interest on the wearer's body being turned on.

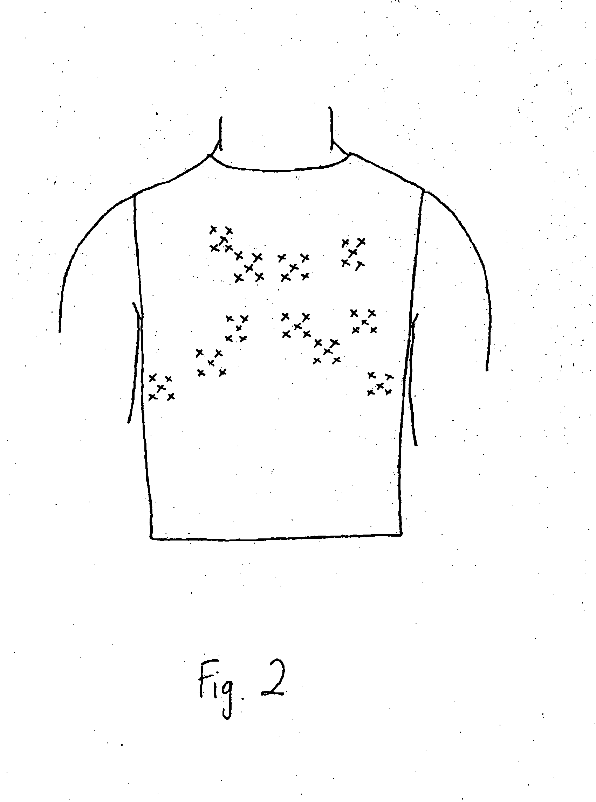

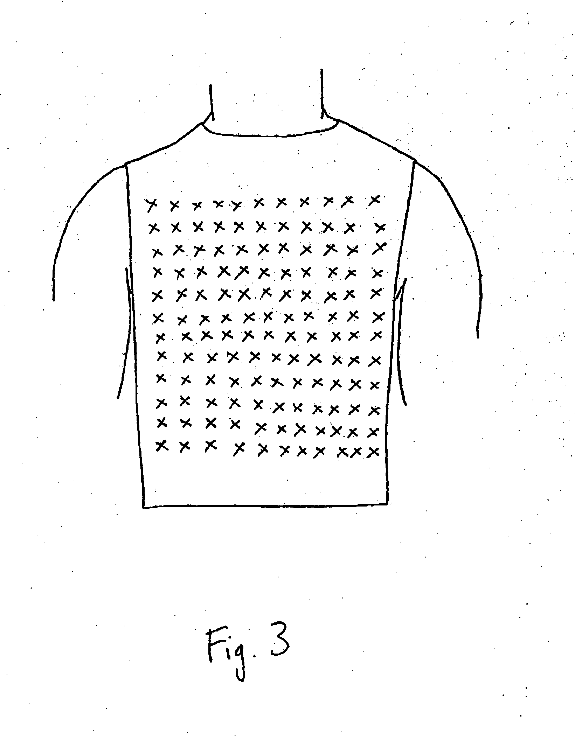

There may be, however, a larger number of sensors provided than auscultation regions. There may, for example, be a plurality of clusters of sensors with a cluster provided at each location on the vest that corresponds generally, in use, to a respective auscultation site on the wearer's chest. Such an arrangement is shown in Figure 2, for the front of the vest, with the locations of the sensors for the anterior auscultation sites marked. A similar arrangement on the rear of the vest to cover or lie next to or adjacent to the posterior auscultation sites may also be provided. Alternatively, there may be an array of evenly spaced sensors, which substantially covers the whole of front and/or rear portions of the vest or substantial contiguous areas of the front and/or rear of the vest, e.g., 30% or more of one or both of said areas or even 50% or more of one or both of said areas. Such an arrangement is shown in Figure 3 for the front of the vest, with the locations of the sensors for the anterior auscultation sites marked. An identical arrangement on the rear of the vest to cover the posterior auscultation sites may also be provided.

The sensor array is not limited to the depicted arrangements. It will be readily appreciated by the skilled person that numerous other arrays are possible.

It is preferable to provide a larger number of sensors, since the exact location of auscultation sites can vary from patient to patient. Furthermore, there may be instances wen the vest is incorrectly positioned on the wearer. In the event a larger number of sensors are provided, a number of the sensors can be shut off with only the sensors that cover or lie next to or adjacent to the areas of interest on the wearer's body being turned on.

The control means may be arranged to control the operation of the sensors. The control means may be arranged to iterate through data acquisition at all the required cardiac and/or pulmonary auscultation sites.

Adaptive beam forming may be used to locate the best signal from an auscultation site. Beam-forming, as will be readily appreciated by the skilled person, is used to control the directionality of the reception or transmission of a signal on a transducer array. In embodiments, beam-forming may be used to correlate sounds detected by different sensors so that the sounds coming from one direction or location may be selected and the sounds coming from other locations may be ignored. Then, the best signals for any particular auscultation site may be selected.

In addition, filtering with matched filter(s) may be applied individually to each sensor, e.g., sequentially, in the local area around an auscultation site to determine which sensor has the best signal for that auscultation site. Then, the sensor(s) determined to be receiving the best signal(s), e.g., best for the diagnosis of ailment(s) or for the monitoring of signal(s) produced by selected organ(s), will be used for medical measurement(s).

Since, with an array of sensors, data may be acquired simultaneously from more than one sensor, source separation may be used to isolate the exact origin of the marker detected. By virtue of such an arrangement it is possible, for example, to detect which part of the heart or lung a detected abnormal sound is coming from.

With simultaneous acquisition from sensors on the front and the back of the vest, it is possible to isolate heart sounds from lung sounds and vice versa.

The sensors may be provided with noise cancelling means, in order to cancel ambient noise, which comes from other sources in the body or in the environment. Such noise cancellation may be achieved using any suitable known filtering methods. Noise cancellation and/or other signal processing may be done in the control means 5 or otherwise.

In addition to the sensors, which are provided to detect body surface vibrations, there may also be sensors provided, which can provide vital or contextual information. Such sensors may include, but are not limited to:
Accelerometers, arranged to detect exertion or activity prior to auscultation in order to put the auscultation readings into context;
Gyroscope sensors or accelerometers, arranged to detect the orientation of the wearer during auscultation i.e. standing, sitting, supine (lying down), arms raised etc. The orientation of the patient is important in auscultation, since sounds detected may be different depending or the position of patient during auscultation;
Sensors arranged to measure the resistivity of the wearer's skin or the heart rate or the wearer, so as to enable the variability wearer's stress levels to be determined. The wearer's stress levels may also be detected from the heart sounds monitored using the sensors, which detect the body surface vibrations; and
Temperature, ECG and blood pressure sensors.

All of the sensors, including both the sensors arranged to detect body surface vibrations and the sensors arranged to provide vital or contextual information are connected to the control means. The control means may, e.g., include one or more of a microprocessor or control circuitry, a power source (battery, or similar) and a wired (serial, USB, Firewire, or similar) or wireless (Bluetooth, Wi-Fi, or similar) connection. The connection allows for the monitored results to be uploaded, either continuously or periodically, to a computer, such as a desktop PC, or other data storage and/or processing means, so that the results may be monitored/reviewed/analysed by a medical professional. The control means may additionally be provided with local storage means (flash memory, tape, or similar) for the backup of sensor data/storage of sensor data between periodical downloads of data from the vest. The vest may be arranged to transmit sensor data to a remote location, for example over the Internet or a cellular or other mobile telephony network.

One particularly advantageous use of the monitoring device of the embodiments is in the diagnosis of sleep apnea. For such a diagnosis, patients have typically been required to spend the night at a sleep clinic with a variety of devices used to diagnosis whether a patients breathing is suspended for 10 seconds or more before awakening. For example, such a diagnosis can be made based on a series of blood oxygen level measurements made via facemasks. However, with the monitoring device of the embodiments, breathing sounds may be isolated to allow for the simple determination of the length of time breathing has been suspended while the wearer is sleeping.

The use of arrays of sensors built into the vest allows for different auscultation sounds from the patients front and back to be automatically acquired without the need for patient co-operation or expert help/interventian.

By virtue of the embodiments, passive, reliable, consistent, continuous monitoring of patients is possible when they are at home, or elsewhere without the aid of a medical professional, even while they sleep.

The description and drawings merely illustrate the principles of the invention. It will thus be appreciated that those skilled in the art will be able to devise various arrangements that, although not explicitly described or shown herein, embody the principles of the invention and are included within its scope. Furthermore, all examples recited herein are principally intended expressly to be only for pedagogical purposes to aid the reader in understanding the principles of the invention and the concepts contributed by the inventor(s) to furthering the art, and are to be construed as being without limitation to such specifically recited examples and conditions. Moreover, all statements herein reciting principles, aspects, and embodiments of the invention, as well as specific examples thereof, are intended to encompass equivalents thereof.

## Claims

1. A monitoring device comprising
a garment (1) having an array of sound or vibration sensors (3) arranged to monitor bodily functions of a wearer of the garment, and control means (5) connected to the sensors,
**characterised by** the control means being arranged to use beam forming to determine which of the sensors are aligned with auscultation sites of the wearer.

2. A monitoring device as claimed in Claim 1, wherein the sensors are arranged to detect body surface vibrations.

3. A monitoring device as claimed in Claim 1 or 2, wherein the array is formed by separate clusters of the sensors, each cluster being located at a location corresponding to a cardiac and/or pulmonary auscultation site of the wearer.

4. A monitoring device as claimed in Claim 3, wherein the sensors are arranged at locations corresponding to the cardiac and pulmonary auscultation sites on the anterior chest and corresponding to the pulmonary auscultation sites on the posterior chest.

5. A monitoring device as claimed in Claim 3, wherein the array comprises more of the sensors than the auscultation sites and wherein the control means is arranged to analyse data acquired at the sensors to determine which of the sensors are aligned with the auscultation sites of the wearer.

6. A monitoring device as claimed in Claim 5, wherein the array covers substantially the entire front and rear portions of the garment.

7. A monitoring device as claimed in Claim 1, wherein the array is formed by a plurality of separated clusters of the sensors, wherein each cluster is located at a respective one of the auscultation locations on the wearer's chest and portions of the garment between the clusters are free of the sensors.

8. A monitoring device as claimed in Claim 1, wherein the control means is arranged to determine which of the sensors are aligned with the auscultation sites using matched filters.

9. A monitoring device as claimed in Claim 1, wherein the control means is arranged to switch off or disregard signals from any of the sensors that are not aligned with the auscultation sites.

10. A monitoring device as claimed in any preceding claim, wherein the sensors comprise microphones, accelerometers, piezoelectric elements, acoustic stethoscopes or electronic stethoscopes.

11. A monitoring device as claimed in Claim 10, wherein the sensors are provided with noise amplification and/or cancellation means.

12. A monitoring device as claimed in any preceding claim, further comprising one or more additional sensors selected from accelerometers, gyroscope sensors, resistance sensors, temperature sensors, ECGs or blood pressure sensors.

13. A monitoring device as claimed in any preceding claim, wherein the garment is a vest.

14. A monitoring system comprising a monitoring device as claimed in any preceding claim, wherein the control means is arranged to be connected to a computer for download of data from the sensors.

15. A method of monitoring bodily functions of the wearer using the monitoring device of any of Claims 1 to 13 or the monitoring system of Claim 14.

## Patentansprüche

1. Überwachungsgerät, bestehend aus
einem Kleidungsstück (1), ausgestattet mit einer Reihe von Sensoren (3), die zur Überwachung der Körperfunktionen des Kleidungsstückträgers ausgelegt sind, und an die Sensoren angeschlossene Steuermittel (5),
**gekennzeichnet dadurch, dass** die Steuermittel ausgelegt sind für den Einsatz von Strahlbildung, um zu ermitteln, welche der Sensoren auf die Auskultationspunkte des Trägers ausgerichtet sind.

2. Überwachungsgerät gemäß Anspruch 1, wobei die Sensoren ausgelegt sind für das Detektieren von Vibrationen der Körperoberfläche.

3. Überwachungsgerät nach Anspruch 1 oder 2, wobei die Reihe von Sensoren durch voneinander getrennte Sensorengruppen gebildet wird und wobei jede Gruppe an einer Stelle platziert ist, die einem Auskultationspunkt des Trägers entspricht.

4. Überwachungsgerät nach Anspruch 3, wobei die Sensoren an Punkten angeordnet sind, die den kardiologischen oder pulmonalen Auskultationspunkten vorne sowie den pulmonalen Auskultationspunkten hinten am Brustkorb entsprechen.

5. Überwachungsgerät nach Anspruch 3, wobei die Reihe von Sensoren mehr Sensoren als Auskultationspunkte umfasst und wobei die Steuermittel so angeordnet sind, dass sie die an den Sensoren erhobenen Daten auswerten können, um zu bestimmen, welche der Sensoren auf die Auskultationspunkte des Trägers ausgerichtet sind.

6. Überwachungsgerät nach Anspruch 5, wobei die Reihe von Sensoren im Wesentlichen die gesamte Vorder- und Rückseite des Kleidungsstücks abdecken.

7. Überwachungsgerät nach Anspruch 1, wobei die Reihe von Sensoren von einer Vielzahl voneinander getrennter Sensorgruppen gebildet wird, wobei jede Gruppe an jeweils einem Auskultationspunkt am Brustkorb des Trägers platziert ist und Bereiche des Kleidungsstücks zwischen den Sensorengruppen keine Sensoren tragen.

8. Überwachungsgerät nach Anspruch 1, wobei die Steuermittel dafür ausgelegt sind, zu bestimmen, welche der Sensoren auf die Auskultationspunkte ausgerichtet sind, wozu angepasste Filter verwendet werden.

9. Überwachungsgerät nach Anspruch 1, wobei die Steuermittel dafür ausgelegt sind, Signale abzuschalten oder außer Acht zu lassen, die von nicht auf die Auskultationspunkte ausgerichteten Sensoren stammen.

10. Überwachungsgerät nach jeglichem der vorgenannten Ansprüche, wobei die Sensoren Mikrofone, Beschleunigungssensoren und piezoelektrische Elemente sowie akustische oder elektronische Stethoskope umfassen.

11. Überwachungsgerät gemäß Anspruch 10, wobei die Sensoren ausgestattet sind mit Mitteln zur Verstärkung oder zum Ausblenden von Geräuschen.

12. Überwachungsgerät nach jeglichem der vorgenannten Ansprüche, weiterhin einen oder mehrere zusätzliche Sensoren des Typs Beschleunigungssensor, Gyroskopsensor, Widerstandssensor, Temperatursensor, EKG-Sensor oder Blutdrucksensor umfassend.

13. Überwachungsgerät nach jeglichem der vorgenannten Ansprüche, wobei es sich bei dem Kleidungsstück um eine Weste handelt.

14. Überwachungssystem, ein Überwachungsgerät nach jeglichem der vorgenannten Ansprüche umfassend, wobei die Steuermittel so ausgelegt sind, dass sie sich für das Herunterladen von Sensorendaten an einen Computer anschließen lassen.

15. Verfahren zur Überwachung der Körperfunktionen des Trägers unter Verwendung des Überwachungsgeräts nach jeglichem der Ansprüche 1 bis 13 oder des Überwachungssystems nach Anspruch 14.

## Revendications

1. Dispositif de monitorage comprenant
un vêtement (1) muni d'un réseau de capteurs (3) de son ou de vibrations adaptés pour surveiller les fonctions corporelles d'un porteur du vêtement et des moyens de commande (5) raccordés aux capteurs,
**caractérisé en ce que** les moyens de commande sont adaptés pour utiliser la mise en forme de faisceau afin de déterminer lesquels des capteurs sont alignés avec des points d'auscultation du porteur.

2. Dispositif de monitorage selon la revendication 1, dans lequel les capteurs sont adaptés pour détecter les vibrations à la surface du corps.

3. Dispositif de monitorage selon la revendication 1 ou 2, dans lequel le réseau est formé par des grappes de capteurs distinctes, chaque grappe étant située à un emplacement correspondant à un point d'auscultation cardiaque et/ou pulmonaire du porteur.

4. Dispositif de monitorage selon la revendication 3, dans lequel les capteurs sont disposés à des emplacements correspondant aux points d'auscultation cardiaque sur le thorax antérieur et correspondant aux points d'auscultation pulmonaire sur le thorax postérieur.

5. Dispositif de monitorage selon la revendication 3, dans lequel le réseau comprend plus de capteurs que de points d'auscultation et dans lequel les moyens de commande sont adaptés pour analyser les données acquises au niveau des capteurs afin de déterminer lesquels des capteurs sont alignés avec les points d'auscultation du porteur.

6. Dispositif de monitorage selon la revendication 5, dans lequel le réseau couvre sensiblement la totalité des portions avant et arrière du vêtement.

7. Dispositif de monitorage selon la revendication 1, dans lequel le réseau est formé par une pluralité de grappes de capteurs distinctes, chaque grappe étant située à un emplacement respectif parmi les emplacements d'auscultation sur le thorax du porteur et les portions de vêtement entre les grappes étant dépourvues de capteurs.

8. Dispositif de monitorage selon la revendication 1, dans lequel les moyens de commande sont adaptés pour déterminer lesquels des capteurs sont alignés avec les points d'auscultation en utilisant des filtres accordés.

9. Dispositif de monitorage selon la revendication 1, dans lequel les moyens de commande sont adaptés pour déconnecter ou ignorer les signaux en provenance de tout capteur qui n'est pas aligné avec les points d'auscultation.

10. Dispositif de monitorage selon l'une quelconque des revendications précédentes, dans lequel les capteurs comprennent des microphones, des accéléromètres, des éléments piézoélectriques, des stéthoscopes acoustiques ou des stéthoscopes électroniques.

11. Dispositif de monitorage selon la revendication 10, dans lequel les capteurs sont dotés de moyens d'amplification et/ou de suppression du bruit.

12. Dispositif de monitorage selon l'une quelconque des revendications précédentes, comprenant en outre un ou plusieurs capteurs supplémentaires choisis parmi les accéléromètres, capteurs gyroscopiques, capteurs résistifs, sondes de température, capteurs d'ECG ou de pression sanguine.

13. Dispositif de monitorage selon l'une quelconque des revendications précédentes, dans lequel le vêtement est une veste.

14. Système de monitorage comprenant un dispositif de monitorage selon l'une quelconque des revendications précédentes, dans lequel les moyens de commande sont adaptés pour être raccordés à un ordinateur pour le téléchargement des données en provenance des capteurs.

15. Procédé de monitorage des fonctions corporelles du porteur en utilisant le dispositif de monitorage selon l'une quelconque des revendications 1 à 13 ou le système de monitorage selon la revendication 14.
